(19) Europäisches Patentamt
European Patent Office
Office européen des brevets

(11) **EP 1 813 675 A1**

(12) **EUROPEAN PATENT APPLICATION**

(43) Date of publication:
**01.08.2007 Bulletin 2007/31**

(51) Int Cl.:
*C12N 15/82* (2006.01)  *A01H 1/04* (2006.01)
*G06T 7/60* (2006.01)

(21) Application number: **07250405.3**

(22) Date of filing: **31.01.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **31.01.2006 US 766605 P**

(71) Applicant: **PIONEER HI-BRED INTERNATIONAL, INC.**
**Johnston, IA 50131 (US)**

(72) Inventors:
 • **Lightner, Jonathan**
  **Des Moines, IA 50312 (US)**
 • **Oliveira, Igor C.**
  **Urbandale, IA 50313 (US)**
 • **Musgrave, Jon**
  **Des Moines, IA 50310 (US)**

(74) Representative: **Bentham, Andrew**
 **J.A. Kemp & Co.**
 **14 South Square**
 **Gray's Inn**
 **London WC1R 5JJ (GB)**

(54) **Method for high throughput transgene function analysis for agronomic traits in maize**

(57)    A method for the rapid evaluation of transgene function in maize plants. The method combines high throughput gene construction methods and high efficiency plant transformation techniques in a specifically developed germplasm. In one as aspect, the method uses quantitative, non-destructive imaging technology applied in a portion or throughout the entire life cycle of a test plant to evaluate agronomic traits of interest in a controlled, statistically relevant greenhouse environment. The method reports transgene function early in the transgenic variety development process, eliminating the need to generate seed necessary for multi-location replicated field trials.

Figure 1

**Description**

**BACKGROUND OF THE INVENTION**

**Field of the Invention**

This patent relates to genetic engineering of plants. More particularly, this patent relates to a high-throughput industrial process for evaluating transgene function in genetically engineered maize.

**Description of the Related Art**

Recent Advances in Plant Transformation

Major advances in plant biotechnology, i.e., technology related to the process of introducing DNA into plant cells, have occurred over the last few years. For example, nucleotide sequencing of the Arabidopsis genome has recently been completed, mapping and sequencing of the rice genome has been completed, and vast quantities of expressed sequence tag information are being obtained from many other plants. This wealth of information provides a powerful tool for the application of genetic engineering methods for improving economically important species.

The primary hurdle in plant biotechnology today is to provide a comprehensive understanding of these nucleotide sequences and the genetic mechanisms controlling plant agronomic traits such as plant growth, development and responses to the environment. The assigning of function to this vast array of gene sequence information will clearly be the most important and perhaps most time consuming step in plant genomics.

Traditional Methods of Evaluating Transgenes

Traditional approaches to assign function to a given set of nucleotide sequences such as expressed sequence tags (ESTs) or various gene/promoter combinations are often not efficient. This is especially true for multi-gene families in which a desired phenotype, such as yield, may be determined by only one or a few of several genes within a gene family, and in which performance of a gene is traditionally evaluated in multi-location field testing. Gene elimination or knockout methods, in which the absence of a gene provides clues to its function, are ineffective for the evaluation of multiple gene families, and provide only indirect evidence of gene function. They are also time consuming, as it takes approximately four generations and up to three years time before an analysis of function can occur, since rounds of backcrossing and selfing are required to fix a given knockout.

Transgene expression for both up and down regulation by transgenics has progressed both in scale and the degree of precision in regulating gene expression. Controlling gene down regulation in transgenic plants has made significant strides with the advent of amplicon, hairpin-loop, and tRNA-like structures which invoke various mechanisms of both transcriptional and post transcription gene silencing for efficient down regulation. Gene up-regulation methods are well known in the art and a variety of promoters are available to produce both ectopic (e.g. 35S and 19S) and targeted (e.g. tissue and temporal promoters) expression patterns (see, e.g., WO 2006/055487 A2). Both up and down regulation approaches to gene function testing are possible in corn, however both suffer from the limitation that, particularly for traits related to yield, the evaluation cycle is slow (in terms of calendar time) and requires large amounts of space to do replicated field studies of yield.

The Problem With Model Systems

Plant "model systems" have been proposed and exploited as a possible solution to the slow evaluation time and large resource requirements (space and manpower) of doing gene testing in maize. A number of systems have been described for transgene function analysis in model plant species, such as Arabidopsis thaliana (see, e.g. WO0183697A2; U.S. Patent Application No. 2004/0128712 A1; and Boyes et al., "Growth Stage-Based Phenotypic Analysis of Arabidopsis: A Model for High Throughput Functional Genomics in Plants", The Plant Cell, Vol. 13, 1499-1510, July 2001), micro tomatoes (Menda et al., "In silico screening of a saturated mutation library of tomato", The Plant Journal (2004); 38, 861-872), and rice. While these systems do generally reduce evaluation time and resource requirements, the biological differences between these model plant species and maize make it difficult to determine the relevance of the model system's results to maize without additional testing and validation in maize itself.

For example, all three of the aforementioned species have a dramatically different photosynthesis mechanism than maize, which has a highly specialized anatomy and metabolism that increases efficiency of $CO_2$ fixation. Arabidopsis and tomato are both dicotyledonous plants while maize is a monocotyledonous species having a fundamentally different seed and plant development pattern. All three of the model species have perfect flowers (flowers that contain both male and female parts), while maize has imperfect flowers (separate pollen producing flowers and seed producing flowers). These and other biological differences render the usefulness of transgenes tested in a model system, particularly those influencing agronomic performance, subject to further testing and validation in maize. Further, the fact that a transgene tested in a model system has no discernable effect is no indication that the same transgene will not have an effect on maize agronomic performance.

Problems Associated With Characterizing New Maize Varieties

There are well-described approaches to characterizing new varieties of maize, whether produced conventionally or via genetic engineering, by their performance in the field. This type of characterization is usually done in multiple locations to resolve environmental effects, and often over multiple growth seasons, which requires significant time, manpower and acreage and other resources. Producing sufficient isogenic plant materials also requires seed increase from the original transformation experiment. Thus the problem remains as to how to rapidly evaluate thousands or tens of thousands of transgenes for function in maize without requiring seed increase and years of testing over multiple generations at multiple field locations.

Objects of the Invention

It is an object of the present invention to provide a method of rapidly evaluating transgene function directly in maize plants and early in the transgenic variety development process, for example, at the T0 and/or T1 generations, without having to generate the seed necessary for multi-location replicated field trials.

It is another object of the invention to provide a method of gene function testing on a much higher scale (1000's to 10,000's of genes per annum), at lower cost, and far more quickly than traditional transgene function testing processes.

It is yet another object of the invention to provide a method of rapid gene function testing that can be done on corn, rather than a model system, so that genes found to be influencing agronomic performance can be immediately introduced into product development, thereby eliminating a costly and failure-prone validation process.

**SUMMARY OF THE INVENTION**

The present invention is a method for the rapid evaluation of transgene function in maize. In one aspect, the method combines high throughput gene construction methods and high efficiency plant transformation techniques in a specifically developed germplasm. In one aspect of the invention, the method uses quantitative, non-destructive imaging technologies applied throughout at least a portion of the target (recipient) plants' life cycle to assess component traits of established relevance to maize agronomic performance in a controlled, statistically relevant, automated greenhouse environment.

In one embodiment, the method involves growing a population of transgenic plants in a controlled greenhouse environment. At selected times the plants are transferred to an imaging analysis area where an imaging analyzer, for example, a quantitative, non-destructive light spectrum digital imaging analyzer, preferably having an instrumental variance below about 5%, takes reflected light images of each plant in the population. The analyzer then analyzes those images to determine a value for a phenotypic parameter of interest for each plant in the population. Various statistical tests can then be applied to the data to determine whether the values of any outliers can be considered attributable to the transgene of interest.

Alternatively, frequency distributions of measurements from each individual quantitative trait can be produced and individuals representing the extreme tails (positive or negative) can be identified as exhibiting potential gene effects. Finally, the mean response of all the events (multiple events for a given gene are created) can be compared to the mean response for the remainder of the transgenics using statistical tests such as students T, TOAST, and others. Those event populations with statistically significant differences in measured parameters indicate strong gene effects and implicate the immediate gene in the control of the measured parameter.

The population of transgenic plants can be a fast cycling uniform maize line to minimize research time. Each plant within the population may be grown in a pot or other container or carrier bearing a machine-readable identification number containing information about the plant's identity and greenhouse location. The identification number can be read before or after the imaging analysis so that the images and the phenotypic parameter data obtained from those images is automatically associated with that plant's identity. The time and date of the individual measurement can also be recorded by the light spectrum digital imaging analyzer so that changes in measured parameters over time (e.g. growth rate) can be determined.

Imaging analysis may be used to determine a number of different phenotypic parameters, including but not limited to leaf angle, anthesis-silking interval (ASI), staygreen ability, early growth rate, total biomass, partial (e.g. leaf, root, stem) biomass and even pollen shed date. Alternatively some of these traits (ASI, anthesis) can be scored with high precision manually (by recording the respective date of anthesis and silking by making daily observations).

The transgenic plants can be grown in a modified randomized block planting pattern to minimize environmental effects on phenotypic parameters. The imaging analyzer may take images of each plant from three or more angles, e.g. under controlled lighting conditions in a housed image analysis area. The plants may be rotated or translated as they pass through the image analysis area and/or alternatively imaged by multiple cameras positioned at multiple angles. For greater efficiency the plants may be transported through the image analysis area by a conveyor system.

A computer may be used to store the data obtained from the imaging analyzer so that the data can be accessed at any time for evaluation and reevaluation.

In another aspect, a control (non-transformed) group of plants is grown among the population of plants transformed with a gene of interest. All plants are then analyzed for a selected phenotypic parameter using the imaging analyzer. The value of the phenotypic parameter for each plant is compared with the values determined for the control plants by the methods described above or other appropriate statistical methods to determine the effect of the gene of interest on the phenotypic parameter.

**BRIEF DESCRIPTION OF THE DRAWINGS**

Figure 1 is a graph of plant volume measured over time for various plant constructs.
Figure 2 is a graph of plant volume measured over time for six plants from the same transgenic event.
Figure 3 is a graph of monitoring % field capacity of soil for a corn water use efficiency assay.

**DETAILED DESCRIPTION OF EXEMPLARY EMBODIMENTS ACCORDING TO THE INVENTION**

The present invention includes a method for the rapid (high throughput) evaluation of transgene function in a maize plant. The method combines high throughput gene construction methods and high efficiency plant transformation techniques in a specifically developed germplasm. In one aspect of the invention, the method uses quantitative, non-destructive imaging technologies applied throughout at least a portion of the entire life cycle of a test plant to evaluate agronomic traits of interest in a controlled, statistically relevant greenhouse environment.

The method reports transgene function early in the transgenic variety development process. That is, the method reports transgene function as early as the T0 and T1 generations without the need to generate the seed necessary for multi-location replicated field trials.

The method also allows transgene function evaluation to be accomplished on a much higher scale - thousands to tens of thousands of genes per year, at dramatically lower cost (because of reduced manpower and field resources), and far more quickly than traditional transgene function testing methods. This can be in part accomplished by utilizing recipient plant cells from a uniform maize line having a short life cycle ("fast cycling"), a reduced size, and high transformation potential.

Further, because the transgene function evaluation is done in corn rather than in model plant species, genes that are discovered to influence agronomic performance can be immediately introduced into product development, thus eliminating the costly and failure-prone gene validation process that would have been required if the transgene had been first tested in a model system.

Entry Vector Construction

Necessary for carrying out the embodiment is the availability of transgene constructs, or entry vectors, suitable for integrating a gene of interest into a recipient plant cell. The entry vector may be constructed using any suitable technology. GATEWAY™ technology commercially offered by Invitrogen Life Technologies of Carlsbad, California USA is one example. The gene of interest may include vectors designed to over-express, to inhibit via loss of function, or to screen for actuation of multiple sequences in plants. Alternatively, the embodiment may be practiced using a library (combination) of different entry vectors containing gene sequences of interest. (See, e.g., U.S. Patent Application Publication No. 2003/0221212, incorporated herein by reference.)

Recipient Plants

The type of recipient plant cells from which the event population is generated is also a feature of the embodiment. This embodiment utilizes recipient plant cells from a uniform maize line having a short life cycle ("fast cycling"), a reduced size, and high transformation potential. Typical of these plant cells for maize are plant cells from any of the publicly available Gaspe Bay Flint (GBF) line varieties. One possible candidate plant line variety is the F1 hybrid of GBF x QTM (Quick Turnaround Maize, a publicly available form of Gaspe Bay Flint selected for growth under greenhouse conditions) disclosed in Tomes et al. U.S. Patent Application Publication No. 2003/0221212. Transgenic plants obtained from this line are of such a reduced size that they can be grown in four inch pots (1/4 the space needed for a normal sized maize plant) and mature in less than 2.5 months. (Traditionally 3.5 months is required to obtain transgenic T0 seed once the transgenic plants are acclimated to the greenhouse.) Another suitable line is a double haploid line of GS3 (a highly transformable line) X Gaspe Flint. Yet another suitable line is a transformable elite inbred line carrying a transgene which causes early flowering, reduced stature, or both. Others, of course, are possible.

Transformation Protocol

Any suitable method may be used to introduce the transgenes into the maize cells including, but not limited to, inoculation type procedures using Agrobacterium based vectors. An Agrobacterium based method may be preferable because of the proven speed with which transformation can occur. Transformation may be performed on immature embryos of the recipient (target) plant using either a vector construct carrying a single or multiple transgenes or a library mixture of vector constructs with sufficient numbers to produce an "event population" which can be grown out and analyzed.

Precision Growth and Plant Tracking

In one aspect of the embodiment, the event population of transgenic (T0) plants resulting from the transformed maize embryos is grown in a controlled greenhouse environment using a modified randomized block design to reduce or eliminate environmental error. A randomized block design is a plant layout in which the experimental plants are divided into groups of, say, thirty plants, referred to as blocks, and each plant is randomly assigned a location with the block.
In one embodiment, twenty-four transformed, experimental plants and six control plants (plants with a set phenotype) (collectively, a "replicate group") are placed in pots which are arranged in an array (a.k.a. a replicate group or block) on a table located inside a greenhouse. Each plant, control or experimental, is randomly assigned to a location with the block which is mapped to a unique, physical greenhouse location as well as to the replicate group. Multiple replicate groups of thirty plants each may be grown in the same greenhouse in a single experiment. The layout (arrangement) of the replicate groups should be determined to minimize space requirements as well as environmental effects within the greenhouse. Such a layout may be referred to as a compressed greenhouse layout.
In another embodiment, all plants in each replicate group are transgenic, experimental plants. The use of control plants in the replicate groups has been found to be an optional feature. Applicants have found that, surprisingly, since most transgenes have little or no phenotypic effect, the mean or median values of the transgenic plants as a whole can serve as a "proxy" for the control group, provided a large enough variety of tested genes are present in a replicate. Thus, a comparison of data for an agronomic trait of interest from a single transformed plant to the mean or median data value for all plants in the event population can be used as a useful indicator of trans gene effect for that trait of interest. This eliminates the need to use valuable greenhouse space to produce a control group of plants. Another alternative to the addition of a specific control group is to identify those transgenic plants that do not express the gene of interest. A variety of techniques such as RT-PCR can be applied to quantitatively assess the expression level of the introduced gene. T0 plants that do not express the transgene can be compared to those which do.
Each plant in the event population is identified and tracked throughout the evaluation process, and the data gathered from that plant can be automatically associated with that plant so that the gathered data can be associated with the transgene carried by the plant. In one embodiment each plant container bears a machine-readable label (such as a Universal Product Code (UPC) bar code) which includes information about the plant identity, which in turn is correlated to a greenhouse location so that data obtained from the plant can be automatically associated with that plant.
Alternatively, any efficient, machine-readable, plant identification system can be used, such as two-dimensional matrix codes or even radio frequency identification tags (RFID) or analogous machine-readable tags or devices in which the data is received and interpreted by a radio frequency receiver/processor. See, e.g., U.S. Published Patent Application No. 2004/0122592, incorporated herein by reference.

Phenotypic Analysis Using Three-Dimensional Imaging

In another aspect, each greenhouse plant in the T0 event population, including any control plants, is analyzed for agronomic characteristics of interest, and the agronomic data for each plant is recorded or stored in a manner so that it is associated with the identifying data (see above) for that plant. If confirmation of a phenotype (gene effect) is necessary this can be accomplished in the T1 generation with a similar experimental design to that described above. It is not necessary to grow further generations (T2, etc.) of plants in the field. Instead, analysis at the phenotypic (e.g. height, maturity, seed set), biochemical (e.g. herbicide resistance) and/or molecular level (direct analysis of gene expression of marker or other genes incorporated into the vector sequence) is performed in the greenhouse on the T0 plants to identify those plants which are relevant to the gene of interest. For example, in Example 1 below the plant event population was analyzed for maximum biomass, one indicator of plant yield.

The T0 plants are analyzed at the phenotypic level using quantitative, non-destructive imaging technology throughout the plant's entire greenhouse life cycle to assess the traits of interest. A digital imaging analyzer is used for automatic multidimensional analyzing of total plants. The imaging may be done inside the greenhouse. In one embodiment, two camera systems, located at the top and side, and an apparatus to rotate the plant, are used to view and image plants from all sides. Images are acquired from the top, front and side of each plant. All three images together provide sufficient information to evaluate the biomass, size and morphology of each plant.

Due to the change in size of the plants from the time the first leaf appears from the soil to the time the plants are at the end of their development, the early stages of plant development are best documented with a higher magnification from the top. This may be accomplished by using a motorized zoom lens system that is fully controlled by the imaging software.

In a single imaging analysis operation, the following events occur: (1) the plant is conveyed inside the analyzer area, rotated 360 degrees so its machine-readable label can be read, and left at rest until its leaves stop moving; (2) side and top images are taken and entered into a database; (3) the plant is rotated 90 degrees, again left at rest until its leaves stop moving, and a different side image is taken, and (4) the plant is transported out of the analyzer.

The system is capable of analyzing many plants, e.g. up to 3,000 plants per day, with each plant being transported from its greenhouse location to the analyzing area and back. For best phenotypic evaluations the plants are allowed at least six hours of darkness per twenty four hour period in order to have a normal day/night cycle, so only eighteen hours are available for plant imaging analysis each day.

Imaging Instrumentation

Any suitable imaging instrumentation may be used, including but not limited to light spectrum digital imaging instrumentation commercially available from LemnaTec GmbH of Wurselen, Germany. In one embodiment the images are taken and analyzed with a LemnaTec Scanalyzer HTS LT-0001-2 having a 1/2" IT Progressive Scan IEE CCD imaging device. The imaging cameras may be equipped with a motor zoom, motor aperture and motor focus. All camera settings may be made using LemnaTec software. Preferably, the instrumental variance of the imaging analyzer is less than about 5% for major components and less than about 10% for minor components.

Software

In one embodiment the imaging analysis system comprises a LemnaTec HTS Bonit software program for color and architecture analysis and a server database for storing data from about 500,000 analyses, including the analysis dates. The original images and the analyzed images are stored together to allow the user to do as much reanalyzing as desired. The database can be connected to the imaging hardware for automatic data collection and storage. A variety of commercially available software systems (e.g. Matlab, others) can be used for quantitative interpretation of the imaging data, and any of these software systems can be applied to the image data set.

Conveyor System

A conveyor system with a plant rotating device may be used to transport the plants to the imaging area and rotate them during imaging. In one embodiment, up to four plants, each with a maximum height of 1.5 m, are loaded onto cars that travel over the circulating conveyor system and through the imaging measurement area. In this embodiment the total footprint of the unit (imaging analyzer and conveyor loop) was about 5 m x 5 m.

Of course, the conveyor system can be enlarged to accommodate more plants at a time. The plants are transported along the conveyor loop to the imaging area and are analyzed for up to 50 seconds per plant. Three views of the plant are taken. The conveyor system, as well as the imaging equipment, should be capable of being used in greenhouse environmental conditions.

Illumination

Any suitable mode of illumination may be used for the image acquisition. In one embodiment a top light above a black background was used. In another embodiment, a combination of top- and backlight using a white background was used. The illuminated area should be housed to ensure constant illumination conditions. The housing should be longer than the measurement area so that constant light conditions prevail without requiring the opening and closing or doors. In yet another variation the illumination can be varied to cause excitation of either transgene (e.g., green fluorescent protein (GFP), red fluorescent protein (RFP)) or endogenous (e.g. Chlorophyll) fluorophores.

Biomass Estimation Based on Three-Dimensional Imaging

For best estimation of biomass the plant images should be taken from at least three axes, preferably the top and two side (sides 1 and 2) views. These images are then analyzed to separate the plant from the background, pot and pollen control bag (if applicable). The volume of the plant can be estimated by the calculation:

$$Volume(voxels) = \sqrt{TopArea(pixels)} \times \sqrt{Side1Area(pixels)} \times \sqrt{Side2Area(pixels)}$$

In the equation above the units of volume and area are "arbitrary units". Arbitrary units are entirely sufficient to detect gene effects on plant size and growth in this system because what is desired is to detect differences (both positive-larger and negative-smaller) from the experimental mean, or control mean. The skilled artisan will recognize that arbitrary units of size (e.g. area) may be trivially converted to physical measurements by the addition of a physical reference to the imaging process. For instance, a physical reference of known area can be included in both top and side imaging processes. Based on the area of these physical references, a conversion factor can be determined to allow conversion from pixels to a unit of area such as square centimeters ($cm^2$). The physical reference may or may not be an independent sample. For instance, the pot, with a known diameter and height, could serve as an adequate physical reference.

Color Classification

The imaging technology may also be used to determine plant color and to assign plant colors to various color classes. The assignment of image colors to color classes is an inherent feature of the LemnaTec software. With other image analysis software systems, color classification may be determined by a variety of computational approaches.
For the determination of plant size and growth parameters the applicants have found that a useful classification scheme is to define a simple color scheme including two or three shades of green and, in addition, a color class for chlorosis, necrosis and bleaching, should these conditions occur. A background color class which includes non-plant colors in the image (for example pot and soil colors) is also used and these pixels are specifically excluded from the determination of size. The plants are analyzed under controlled constant illumination so that any change within one plant over time, or between plants or different batches of plants (e.g. seasonal differences) can be quantified.
In addition to its usefulness in determining plant size growth, color classification can be used to assess other yield component traits. For these other yield component traits additional color classification schemes may be used. For instance, the trait known as "staygreen", which has been associated with improvements in yield, may be assessed by a color classification that separates shades of green from shades of yellow and brown (which are indicative of senescing tissues). By applying this color classification to images taken toward the end of the T0 or T1 plants' life cycle, plants that have increased amounts of green colors relative to yellow and brown colors (expressed, for instance, as Green/Yellow Ratio) may be identified. Plants with a significant difference in this Green/Yellow ratio can be identified as carrying transgenes which impact this important agronomic trait.
The skilled plant biologist will recognize that other plant colors arise which can indicate plant health or stress response (for instance anthocyanins), and that other color classification schemes can provide further measures of gene action in traits related to these responses.

Plant Architecture Analysis

Transgenes which modify plant architecture parameters may also be identified using one or more aspects of the present invention, including such parameters as maximum height and width, internodal distances, angle between leaves and stem, number of leaves starting at nodes and leaf length. The LemnaTec system software may be used to determine plant architecture as follows. The plant is reduced to its main geometric architecture in a first imaging step and then, based on this image, parameterized identification of the different architecture parameters can be performed. Transgenes that modify any of these architecture parameters either singly or in combination can be identified by applying the statistical approaches previously described.

Pollen Shed Date

Pollen shed date is an important parameter to be analyzed in a transformed plant, and may be determined by the first appearance on the plant of an active male flower. To find the male flower object, the upper end of the stem is classified by color to detect yellow or violet anthers. This color classification analysis is then used to define an active flower, which in turn can be used to calculate pollen shed date.

Alternatively, pollen shed date and other easily visually detected plant attributes (e.g. pollination date, first silk date) can be recorded by the personnel responsible for performing plant care. To maximize data integrity and process efficiency this data is tracked by utilizing the same barcodes utilized by the LemnaTec light spectrum digital analyzing device. A computer with a barcode reader, a palm device, or a notebook PC may be used for ease of data capture recording time of observation, plant identifier, and the operator who captured the data.

Orientation of the Plants

Mature maize plants grown at densities approximating commercial planting often have a planar architecture. That is, the plant has a clearly discernable broad side, and a narrow side. Applicants have found that, to obtain the most reliable plant architecture data, the image of the plant from the broadside is of highest importance. To achieve reproducible information it is desired to assign to each plant a well-defined basic orientation to obtain the maximum difference between the broadside and edgewise images. Otherwise many of the length measurements will not be reproducible because they vary greatly due to orientation preference of the leaves. It is suggested that the top image be used to determine the main axis of the plant, and an additional rotating device be included to turn the plant to the appropriate orientation prior to starting the main image acquisition.

**EXAMPLES**

The following example(s) are intended to illustrate aspects of the invention described herein but are not intended to limit the invention in any way.

Example 1 - Assessing the Effect of a Transgene on Maximum Plant Biomass Without The Use of a Non-Transgenic Control Group

Yield in corn is commonly thought to be a function of a number of agronomic traits, including leaf angle, anthesis-silking interval (ASI), staygreen ability, early growth rate, and total biomass (plant volume). The present invention takes advantage of the fact that some of these traits are deployed (exhibited) early in a plant's life cycle. For example, maximum plant biomass is one determining factor for yield. Applicants determined plant volume in multiple replications using three-dimensional imaging technology to assess the effect of transgenes on corn yield.

Assessing Instrumental Variance

The instrumental variance of a LemnaTec Scanalyzer HTS LT-0001-2 was determined by analyzing the biomass of a single plant four times. The variability of the total voxel number from image to image was found to be about 2-3 % of total plant volume. Thus, the precision of the instrument was deemed to be adequate for performing bioassays.

Analysis of Maximum Biomass in an Event Population of Transformed Corn

T0 plants grown from the T0 maize germplasm that had been transformed with a gene of interest (GOI) were grown at the same time and in the same controlled greenhouse environment using a randomized complete block design to reduce or eliminate environmental error. Each plant was mapped to a unique, physical greenhouse location as well as to a replicate group. Multiple replicate groups of 30 plants were grown in the same greenhouse.

The T0 plants were evaluated using the same three-dimensional digital imaging analyzer (a LemnaTec Scanalyzer HTS LT-0001-2). Biomass data was obtained over the plant's entire life cycle.

The sixteen transgene constructs that were evaluated for maximum plant biomass are listed in Table 1. Each of the sixteen constructs was used to create up to ten T0 transgenic events (see Table 1) which were then grown and evaluated in a modified randomized complete block design as described above.

TABLE 1

| Transgenic Events Per Construct | |
| --- | --- |
| Expt Name | GN00061.03.21.05 - |
| Series | 1 |
| | |
| | |
| Construct ID | Total Events |
| PHP24877 | 10 |
| PHP24881 | 10 |
| PHP24885 | 10 |
| PHP24889 | 10 |
| PHP24892 | 9 |
| PHP24893 | 10 |
| PHP24897 | 10 |
| PHP24901 | 10 |
| PHP24905 | 10 |
| PHP24908 | 10 |
| PHP24914 | 10 |
| PHP24917 | 10 |
| PHP24920 | 8 |
| PHP24923 | 5 |
| PHP24926 | 8 |
| PHP24929 | 7 |
| Grand Total | 14 |

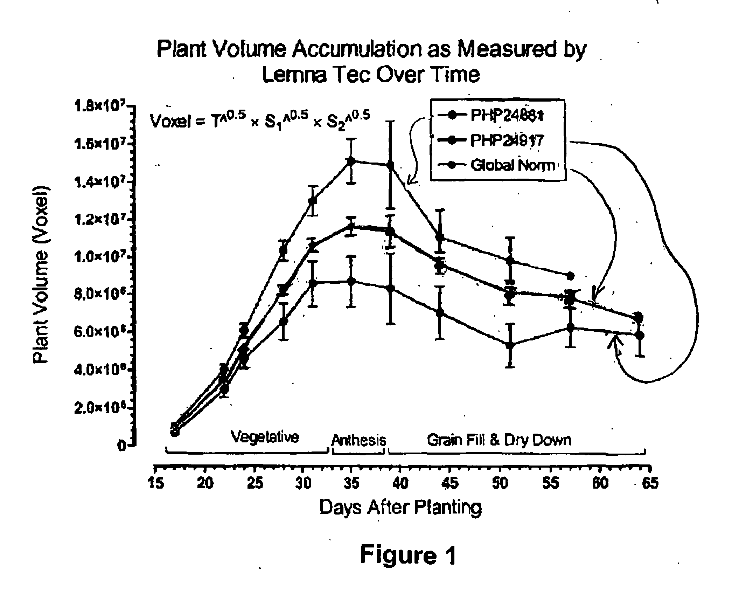

Each event is represented by a single plant which was uniquely identified by a barcode method as described above. Each plant was assayed throughout the T1 life cycle using the LemnaTec Scanalyzer HTS LT-0001-2. The plant surface areas determined for the two side and top views were used to estimate aerial biomass in arbitrary units of voxels. By plotting these biomass (voxel) determinations over time a growth curve is obtained and the maximum biomass is extracted, as shown in Figure 1. Rather than compare maximum biomass to a control group of plants, in this experiment each construct was compared to all the other constructs in the group. Events were considered replications of their respective constructs and are reported as such in Figure 1. The mean performance of all constructs in the experiment (the "Global Norm") is shown as well.

By treating the events as replicates for the construct it is possible to use the T0 plant volume data of Figure 1 to perform statistical tests (for example, t-test, f-test, TOAST and others). Table 2 presents the t-test data for the maximum biomass values for T0 plants arising from all the events of construct PHP24881 versus the maximum biomass for all T0 plants of the other transgenic events.

TABLE 2

| Statistical Comparison of Maximum Plant Volume (Biomass) Between PHP24881 and All Other Transgenic Events | | |
| --- | --- | --- |
| (t-Test: Two-Sample Assuming Unequal Variances) | | |
| | *All Others* | *PHP24881* |
| Mean Maximum Plant Volume | 12250928 | 15379234 |
| Sample Variance | 1.473E+13 | 1.524E+13 |
| Observations | 137 | 10 |
| | | |
| Hypothesized Mean Difference | 0 | |
| Df | 10 | |
| t Stat | -2.449 | |

(continued)

| Statistical Comparison of Maximum Plant Volume (Biomass) Between PHP24881 and All Other Transgenic Events | | |
|---|---|---|
| (t-Test: Two-Sample Assuming Unequal Variances) | | |
| | All Others | PHP24881 |
| P(T<=t) one-tail | 0.017 | |
| t Critical one-tail | 1.812 | |
| P(T<=t) two-tail | 0.034 | |
| t Critical two-tail | 2.228 | |

As shown in Table 2, the calculated t value (t Stat) of 2.449 is greater than the critical t value (two tailed) of 2.228. From this we conclude that the mean maximum biomass for the PHP24881 construct is significantly higher than the mean maximum for the rest of the event population at p = 0.034.

An identical t-test (assuming unequal variance) comparing the maximum biomass for all the events of construct PHP24917 versus the maximum biomass for all of the other transgenic events in the experiment shows that construct PHP24917 produces a highly statistically significant decrease in biomass, as shown in Table 3.

TABLE 3

| Statistical Comparison of Maximum Plant Volume (Biomass) Between PHP24917 and All Other Transgenic Events | | |
|---|---|---|
| (t-Test: Two-Sample Assuming Unequal Variances) | | |
| | All others | PHP24917 |
| Mean Maximum Plant Volume | 12693185 | 9320312 |
| Variance | 1.47E+13 | 1.38E+13 |
| Observations | 137 | 10 |
| Hypothesized Mean Difference | 0 | |
| Df | 10 | |
| t Stat | 2.768169 | |
| P(T<=t) one-tail | 0.009925 | |
| t Critical one-tail | 1.812461 | |
| P(T<=t) two-tail | 0.01985 | |
| t Critical two-tail | 2.228139 | |

The skilled artisan will recognize that such statistical analysis can be most efficiently accomplished by automated software processing and that a variety of different statistical approaches, both hypothesis testing and descriptive statistics, can be successfully used to identify outliers which, if they are associated with a particular gene construct, are indicative of gene function. This example demonstrates that from a single generation of data (T0) it is clearly possible to identify both best performing and worst performing transgenes if one is interested in increasing plant productivity.

Example 2 - Assessing the Effect of a Transgene on Flowering Time Using a Control Group

A replicate group of 30 plants was scored (analyzed) for flowering time based on determination of date of anthesis using the statistical methods described above. Data was obtained for a block of 28 plants, consisting of T0 (first generation) plants from 16 different GOI constructs having either one or two transgenic events and four non-transformed, control plants. The date of anthesis was expressed as "days to shed", that is, the number of experiment days that passed before anthesis was observed. For these purposes the experiment was considered initiated when seedlings were transferred to the greenhouse after acclimation of the T0 seedlings in soil. The mean and standard deviation of "days to shed" was calculated for the control plants (6.75 days and 0.5 days respectively) and used to establish a threshold value for "early flowering," which was two standard deviations below the mean (Early Flowering Threshold = 6.75 - (2 * 0.5) = 5.75 Days). Each measured value for a transgenic (T0) plant was compared to the threshold value with a simple Boolean expression and any plants with "days to shed" below the threshold value were flagged. The results are shown in Table 4.

TABLE 4

Comparison of Observed Flowering Time (Days to Shed) For Transgenic Plants With Threshold Flowering Time Established by Control Plants

| GOI Construct ID | PLANT ID | Measured "Days to shed" less than Threshold |
|---|---|---|
| PHP24877 | 47613637 | 1 |
| PHP24877 | 47613638 | 1 |
| PHP24881 | 47637921 | 0 |
| PHP24881 | 47637922 | 0 |
| PHP24885 | 47613646 | 0 |
| PHP24889 | 47637938 | 0 |
| PHP24889 | 47637939 | 0 |
| PHP24892 | 47613542 | 0 |
| PHP24892 | 47613543 | 0 |
| PHP24893 | 47613656 | 0 |
| PHP24897 | 47637962 | 0 |
| PHP24901 | 47613665 | 0 |
| PHP24901 | 47613666 | 0 |
| PHP24905 | 47637976 | 0 |
| PHP24908 | 47613554 | 0 |
| PHP24908 | 47613555 | 0 |
| PHP24914 | 47613562 | 0 |
| PHP24914 | 47613563 | 0 |
| PHP24917 | 47637986 | 0 |
| PHP24920 | 47613573 | 0 |
| PHP24923 | 47638088 | 0 |
| PHP24926 | 47613616 | 0 |
| PHP24926 | 47613617 | 0 |
| PHP24929 | 47638107 | 0 |
| Control | 47437776 | 0 |
| Control | 47437777 | 0 |
| Control | 47437779 | 0 |
| Control | 47437780 | 0 |

As shown in Table 4, only two of the 24 transgenic plants in this experiment exhibited an early flowering time compared to the established threshold. If flowering time as described by "days to shed" is a normally distributed data set, plants that exceed the 2 standard deviation threshold for early flowering time should occur by chance only 5% of the time. Because both of these early flowering observations occurred in transgenics carrying the same GOI construct (PHP24877), it may be concluded that the GOI construct PHP24877 causes a significant change in flowering time.

Example 3 - Quantitative Analysis of T1 Plants to Identify Transgenes That Confer Increased Biomass Accumulation

In order to achieve higher quantitative phenotypic resolution, T1 progeny of T0 plants can be obtained by pollinating the T0 plants with a recurrent parent (for example: Gaspe Flint mentioned above, or another desired corn variety, optimally a small stature rapid cycling variety). T0 plants obtained from construct PHP22760 were pollinated with GF-3, an inbred developed from Gaspe Bay Flint. T1 seeds from four transgenic events (Table 5) were sown and assayed for the presence of the transgene. From each transgenic event, T1 plants positive for transgene carrying and negative for null segregants were identified by scoring for the presence of the marker gene. Because the T0 plants (hemizygous) were outcrossed, the transgene segregates 1:1 in the T1 progeny. Table 5 indicates the number of trait positive and null segregants obtained for each of the events evaluated.

TABLE 5

Individual Transgenic Events of PHP22760 Selected for T1 Analysis

| Construct | Event Name | Transgene + | Null Segregants (Transgene -) |
|---|---|---|---|
| PHP22760 | EA1889.045.1.2 | 4 | 2 |
| PHP22760 | EA1889.045.1.6 | 3 | 3 |
| PHP22760 | EA1889.045.1.7 | 3 | 3 |
| PHP22760 | EA1889.045.1.10 | 4 | 2 |

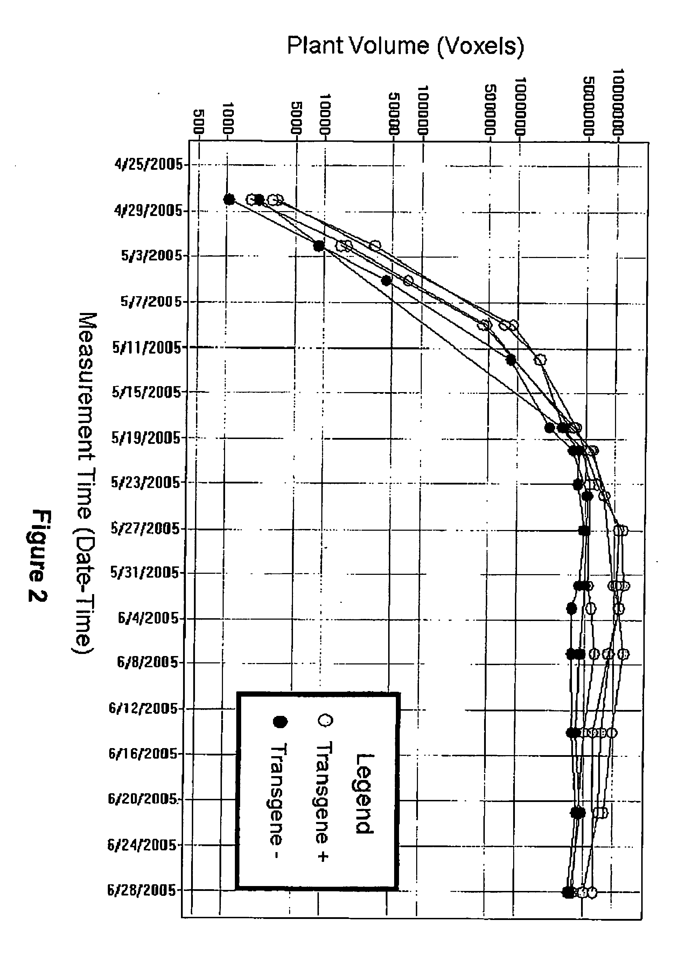

Plant growth and development was quantitatively evaluated using the LemnaTec Scanalyzer and Software as detailed in Example 1. Figure 2 shows growth curves obtained for six plants from event EA1889.045.1.2. A clear difference is observed in the peak of the biomass accumulation of the transgene carrying (transgene +) versus the null segregants (transgene - ) from this transgenic event.

The maximum biomass observed was extracted from the growth data of each of the 24 individual plants evaluated and used to make a statistical comparison between trait positive and trait negative segregants in each event. One-tailed T-tests (assuming unequal variance) were performed to determine the significance level of the differences observed between the mean maximum biomass observed in trait positive and trait negative lines. Table 6 shows the results of these T-tests evaluating all four events together, and the individual events separately.

TABLE 6

P Values Determined for One-Tailed T-tests Tested Group (+ vs. null

| segregants) | P(T<=t) one-tail |
|---|---|
| All 4 events | 0.009 |
| EA1889.045.1.2 | 0.014 |
| EA 1889.045.1.6 | 0.469 |
| EA1889.045.1.7 | 0.001 |
| EA1889.045.1.10 | 0.221 |

Table 6 shows that a highly statistically significant difference in maximum biomass is observed for the entire group of 4 events from plasmid PHP22760, but by evaluating the events individually it is clear that most of this effect is contributed by two events, EA1889.045.1.2 and EA1889.045.1.7. By analyzing segregants within each transgenic event at the T1 generation, additional information is gained as to the range of effects that may be produced by the tested gene.

Example 4 - Identification of Transgenes that Modify Flowering Time Without the Use of a Control Group.

All the plants were scored (analyzed) for flowering time based on the date of anthesis, which was expressed as "days to shed" and was described in Example 2. All the transgenic T0 plants on one greenhouse table can be considered as one analysis unit (one "rep"). The mean (Rep Mean) and standard deviation (Rep Stdev) of flowering time were calculated for all the T0 experimental materials in each rep. A Z-Score was calculated for each transgenic event to determine the distance of the individual event result from the Rep Mean (early or late) based on the standard deviation for the rep. This flowering Z-score is an expression of the distance of an individual result from the mean of a population as the number of positive or negative standard deviations. In this Example, it is (Event Result-Rep Mean) /Rep Stdev. We can identify the leads based on a certain threshold. For example, we were able to identify six constructs from a set of 800 evaluated transgenes when we set the threshold as "all the constructs which have at least two events with Z score >=2 (late flowering) or <=-2 (early flowering)". The results are shown in Table 7.

As shown in Table 7, three constructs PHP27500, PHP23291, PHP23648 have at least two events which have Z Score <=-2. In addition, other events in those constructs also have the tendency of low Z Score, for example, four events from PHP27500 have Z Score <=-1 and >-2. Meanwhile, three constructs PHP26747, PHP26196 and PHP24201 have at least two events which have Z Score >=2. If flowering time as described by "days to shed" is a normally distributed data set, plants which have Z score >=2 or <=-2 for flowering time should occur by chance only 5% of the time. It can be concluded that we are able to identify constructs that significantly modify flowing time by mining the available dataset with thresholds defined by the experimental materials on average rather than using thresholds established with control plants.

TABLE 7

Observed Flowering Time (Days to Shed) of Transgenic Event and Its Z Score comparing to Rep Mean

| Construct ID | Event Name | Event Result | Rep Mean | Rep Stdev | Event Z Score |
|---|---|---|---|---|---|
| PHP27500 | EA1909.462.1.1 | 29 | 34.9 | 2.2 | -2.7 |
| | EA1909.462.1.2 | 30 | 34.6 | 2.9 | -1.6 |
| | EA1909.462.1.3 | 29 | 36.3 | 4 | -1.8 |
| | EA1909.462.1.4 | 30 | 36.8 | 3.9 | -1.7 |
| | EA1909.462.1.5 | 32 | 35.9 | 3.4 | -1.1 |
| | EA1909.462.1.6 | 30 | 35.5 | 2.4 | -2.3 |
| | EA1909.462.1.7 | 34 | 36.4 | 2.5 | -0.9 |
| | EA1909.462.1.8 | No Data | 36.4 | 2.5 | - |
| | EA1909.462.1.9 | No Data | 36.5 | 1.2 | - |
| | EA1909.462.1.10 | No Data | 35.8 | 1.5 | - |
| PHP27500 | 2 events with Z Score <=-2 | | | | |
| PHP23291 | EA1909.016.1.1 | 30 | 34.4 | 2.5 | -1.8 |
| | EA1909.195.1.1 | 32 | 35.9 | 4.1 | -1 |
| | EA1909.195.1.2 | 35 | 35.9 | 4.1 | -0.2 |
| | EA1909.195.1.3 | 35 | 35 | 1.4 | 0 |
| | EA1909.195.1.4 | 31 | 35 | 1.4 | -2.8 |
| | EA1909.195.1.5 | 31 | 34.5 | 1.7 | -2 |
| | EA1909.195.1.6 | 35 | 34.5 | 1.7 | 0.3 |
| | EA1909.195.1.7 | 32 | 34.5 | 1.7 | -1.4 |
| | EA1909.195.1.8 | 32 | 35.1 | 2 | -1.5 |
| | EA1909.195.1.9 | 32 | 35.1 | 2 | -1.5 |
| | EA1909.195.1.10 | 35 | 35.1 | 2 | 0 |
| PHP23291 | 2 events with Z Score <=-2 | | | | |
| PHP23648 | EA1890.080.1.1 | 33 | 35.8 | 3.4 | -0.8 |
| | EA1890.080.1.2 | 30 | 34.9 | 2.1 | -2.3 |
| | EA1890.080.1.3 | No Data | 36.2 | 3.4 | - |
| | EA1890.080.1.4 | 32 | 35.1 | 1.7 | -1.8 |
| | EA1890.080.1.5 | 26 | 34.2 | 3.1 | -2.7 |
| | EA1890.080.1.6 | 26 | 33.9 | 2.7 | -2.9 |
| | EA1890.080.1.7 | 31 | 34.7 | 2.2 | -1.7 |
| | EA1890.080.1.8 | 35 | 35.7 | 1.9 | -0.4 |
| | EA1890.080.1.9 | No Data | 35.4 | 3 | - |
| | EA1890.080.1.10 | No Data | 35.1 | 2 | - |
| PHP23648 | 3 events with Z Score <=-2 | | | | |
| PHP26747 | EA1890.389.1.1 | 36 | 37.6 | 3.1 | -0.5 |
| | EA1890.389.1.2 | 36 | 37.6 | 3.1 | -0.5 |
| | EA1890.389.1.3 | 57 | 39.4 | 6.2 | 2.8 |
| | EA1890.389.1.4 | 59 | 39.4 | 6.2 | 3.1 |
| | EA1890.389.1.5 | 63 | 39.8 | 7.5 | 3.1 |
| | EA1890.389.1.6 | 60 | 39.8 | 7.5 | 2.7 |
| | EA1890.389.1.7 | 61 | 41.1 | 7.5 | 2.7 |
| | EA1890.389.1.8 | 57 | 41.1 | 7.5 | 2.1 |
| | EA1890.389.1.9 | 36 | 37.4 | 2.7 | -0.5 |
| | EA1890.389.1.10 | No Data | 37.4 | 2.7 | - |
| PHP26747 | 6 events with Z Score >=2 | | | | |
| PHP26196 | EA1890.331.1.1 | 40 | 34.4 | 2.3 | 2.4 |
| | EA1890.331.1.2 | 37 | 34.4 | 2.3 | 1.1 |
| | EA1890.331.1.3 | 34 | 34.6 | 1.6 | -0.4 |

(continued)

Observed Flowering Time (Days to Shed) of Transgenic Event and Its Z Score comparing to Rep Mean

| Construct ID | Event Name | Event Result | Rep Mean | Rep Stdev | Event Z Score |
|---|---|---|---|---|---|
| | EA1890.331.1.4 | 34 | 34.6 | 1.6 | -0.4 |
| | EA1890.331.1.5 | 35 | 34.3 | 2.4 | 0.3 |
| | EA1890.331.1.6 | 34 | 34 | 1.4 | 0 |
| | EA1890.331.1.7 | 35 | 34.4 | 1.6 | 0.4 |
| | EA1890.331.1.8 | 41 | 33.8 | 2.8 | 2.6 |
| | EA1890.331.1.9 | 34 | 33.8 | 1.3 | 0.2 |
| | EA1890.331.1.10 | 35 | 33.8 | 1.3 | 0.9 |
| PHP16196 | 2 events with Z Score >=2 | | | | |
| PHP24201 | EA1890.153.1.1 | 37 | 37.4 | 2.5 | -0.1 |
| | EA1890.153.1.2 | 43 | 37.4 | 2.5 | 2.3 |
| | EA1890.153.1.3 | 36 | 37.4 | 2.5 | -0.5 |
| | EA1890.153.1.4 | 42 | 36.4 | 2 | 2.8 |
| | EA1890.153.1.5 | 38 | 36.4 | 2 | 0.8 |
| | EA1890.153.1.6 | 39 | 35.6 | 2.7 | 1.3 |
| | EA1890.153.1.7 | 38 | 35.6 | 2.7 | 0.9 |
| | EA1890.153.1.8 | 38 | 36.9 | 1.6 | 0.7 |
| | EA1890.153.1.9 | 37 | 36.9 | 1.6 | 0.1 |
| | EA1890.153.1.10 | 37 | 36.9 | 1.6 | 0.1 |
| PHP24201 | 2 events with Z Score >=2 | | | | |

Example 5 - Improvement of Gene Selection Using Transgene Expression Data Produced at T0 Generation

In addition to the plant performance measurements made with imaging technology or observation as previously described, further utility from the process described can be gained by analyzing the expression of the transgene of interest. Each T0 transgenic plant is analyzed for expression of transgene (target gene) using a high throughput approach, such as quantitative real time RT-PCR. Relative expression of transgene can be calculated by comparing the expression level of the target gene to an endogenous reference gene (e.g. Actin gene) and/or comparing transgenic plant to a control sample, such as a pool of non-transgenic plants or pool of transgenic plants that do not carry the target transgene. Expression data are recorded and associated with the results obtained from other phenotypic, biochemical, molecular, and/or bioassay (e.g. insect resistance) analysis.

T0 events obtained from constructs PHP26201, PHP26225, PHP26213 were assayed for European corn borer resistance and relative expression level of transgene. European corn borers are important agronomic pests of corn. Their feeding causes economic damage and consequently has been controlled both genetically and with crop protection chemicals. The feeding of corn borers can be tested with a leaf disc bioassay. Leaf clippings from each T0 plant were infected with approximately 5-10 neonate larvae. Two days after the infection, the observations were recorded as: "Positive" or "Negative". For transgene expression, an assay was developed based on the common sequence region of the target transgenes from those constructs and relative expression level to Actin was calculated for each event. The results are shown in Table 8.

As showed in Table 8, we were not able to detect transcript in the events carrying PHP26201; meanwhile the same assay showed high levels of transcript (1-585.81x Actin) in the events produced with PHP26225 and PHP26213, indicating the expression level difference is not caused by the assay. The negative bioassay results of T0 events from PHP26201 result from the absence of the expression of the target transgene in the events. The target gene in PHP26213 can be expressed well in the T0 events (1-210.87x Actin) but the transgene does not have the expected effects (positive bioassay result). All the events from PHP26225 have positive bioassay results and good expression level of transgene. The results suggest the target gene in PHP26225 is a good lead for European corn borer resistance. Optimal transgenes are those which are expressed well, as detected by qRT-PCR and have bioactivity.

This Example shows that the ability to select genes is clearly improved by using transgene expression at T0 events. In many cases bioassays are very labor intensive and rate limiting. In this Example, by using the expression level of the transgene as a pre-screen, one transgene construct, PHP26201, could have been eliminated from bioassay testing. Eliminating this construct based on the expression data would have resulted in an approximately 30% improvement in the efficiency of the work. Thus, adding expression analysis at the T0 level further improves the efficiency of the process.

TABLE 8

| Transgene expression and bioassay results | | | |
|---|---|---|---|
| Construct | Plant ID | Bioassay | Relative Expression of Transgene |
| PHP26201 | 51745608 | Negative | 0.00 |
| PHP26201 | 51745609 | Negative | 0.00 |
| PHP26201 | 51745610 | Negative | 0.00 |
| PHP26201 | 51745611 | Negative | 0.00 |
| PHP26201 | 51745612 | Negative | 0.00 |
| PHP26201 | 51745613 | Negative | 0.00 |
| PHP26201 | 51745614 | Negative | 0.00 |
| PHP26201 | 51745615 | Negative | 0.00 |
| PHP26201 | 51745616 | Negative | 0.00 |
| PHP26201 | 51745617 | Negative | 0.00 |
| PHP26225 | 51745668 | Positive | 82.54 |
| PHP26225 | 51745669 | Positive | 25.90 |
| PHP26225 | 51745670 | Positive | 53.86 |
| PHP26225 | 51745671 | Positive | 52.57 |
| PHP26225 | 51745672 | Positive | 148.90 |
| PHP26225 | 51745673 | Positive | 585.81 |
| PHP26225 | 51745674 | Positive | 20.34 |
| PHP26225 | 51745675 | Positive | 30.90 |
| PHP26225 | 51745676 | Positive | 58.45 |
| PHP26225 | 51745677 | Positive | 406.16 |
| PHP26213 | 51884376 | Negative | 1.00 |
| PHP26213 | 51884377 | Negative | 31.87 |
| PHP26213 | 51884378 | Negative | 46.63 |
| PHP26213 | 51884379 | Negative | 1.00 |
| PHP26213 | 51884380 | Negative | 16.60 |
| PHP26213 | 51884381 | Negative | 210.87 |
| PHP26213 | 51884382 | Negative | 184.12 |
| PHP26213 | 51884383 | Negative | 27.60 |
| PHP26213 | 51884384 | Negative | 23.45 |

Example 6 - Assessing the Effect of a Transgene on Nitrogen Use Efficiency at T1 Generation

Functional testing of transgenes that improve agronomic traits such as nitrogen use efficiency (NUE) can be evaluated in rapid (high throughput) assays carried out in controlled, statistically relevant, semi-automated greenhouse environments. The present example takes advantage of the fact that maize plants exhibit several physiological and phenotypic traits early on in development under limiting nitrogen conditions. Nitrogen use efficiency is of sufficient agronomic interest because high corn yields in current agricultural practice require high levels of applied chemical nitrogen fertilizer. Both the application of the fertilizer and the cost of the fertilizer are presently high. Transgenes which could increase the ability of corn plants to use applied nitrogen would allow either high yields at normal nitrogen application levels or normal yields at reduced levels of applied nitrogen and would have obvious economic value. Improvements in nitrogen use efficiency can be manifest in many ways which can include, but are not limited to, increased biomass accumulation and greenness; two traits that correlate well with the plant's ability to respond to the amount of nitrogen in the environment. Identifying transgenes which have a positive impact on NUE in the field is especially challenging because field soils will often contain organic nitrogen which becomes available in an uncontrolled way as organic matter decays. Variation across the field in both organic nitrogen and applied nitrogen requires that field NUE studies be large and well replicated. Finally, Nitrogen gas in the air can be fixed and made available by lightning strikes during thunderstorms. Therefore, available nitrogen levels in field soils are extremely hard to control and, thus, useful transgenes can only be detected with high levels of replication. These levels require large amounts of seed which can only be produced through several generations.

Using three dimensional imaging technologies, the effect of various transgenes on nitrogen use efficiency can be assessed early on in the transgenic variety development process (i.e. T1 generation). This technology is able to identify genes that influence agronomic performance such as plants with enhanced traits prior to field testing. Enhancements in nitrogen use efficiency can be measured by monitoring changes in plant growth and chlorophyll content under limiting and/or sufficient nitrogen containing environments using, e.g., a LemnaTec Scanalyzer HTS LT-0001-2. Plant growth is assessed by capturing plant volume data throughout the duration of the assay as described in Example 1. In addition, the color information captured by the imaging system is used to analyze the accumulation of specific color groups over time. For detecting NUE, four color classes are defined using the LemnaTec software; green, light green, yellow, and magenta. More complex color classification schemes may be developed, however for NUE, this is one treatment that discriminates well between limiting and non-limiting plant growth. Using the LemnaTec software, all pixels in the plant image are assigned to one of these four classes or to the background. NUE is assessed by comparing the percentage of green colors with percentage of other colors such as light green and yellow. A balanced, incomplete, randomized block design with stationary blocks within treatments is used in order to minimize spatial variation. The experiment is blocked by treatment and randomized by events and replicates.

T1 plants are generated by crossing individual T0 transfromant, hemizygous plants with a recurrent male parent; optimally a small stature, rapid cycling inbred variety. The skilled artisan will recognize that T0 transformants may also be self-pollinated, or pollinated with other varieties. The applicants have found that the use of a recurrent, inbred parent, of rapid cycling characteristics, is presently most optimal for the conduct of this assay with minimum plant numbers. Each T1 plant is grown in a classic 200 pot (volume equivalent to 1.7L) that contains a bar coded label with information about the plant's genetic identity, planting date and greenhouse location. The planting density is 8.5" center to center within a row and 9" center to center between rows. T1 seeds are sown in Turface (commercially available from Profile Products LLC, Buffalo Grove, IL, USA), a baked clay-like material with high cationic exchange properties, at a uniform depth of 1.5" from the surface of the growing medium. Seedlings segregating 1:1 are assayed for the presence of a marker gene that is contained within the T-DNA in order to identify transgene positive versus null segregants (outlined in Example 3). Twenty plants consisting of 10 positive plants and 10 negative plants for each event are planted.

Two treatments are applied during the NUE Assay; a limiting nitrogen-containing environment and a nitrogen-containing environment that is sufficient or more optimal for plant growth. Nutrients are applied daily and include a modified Hoagland's nutrient solution (Table 1). The low nitrogen treatment contains 1mM $KNO_3$ and the more optimal nitrogen treatment contains 6.5mM $KNO_3$. Both nitrogen containing nutrient solutions are maintained as 20X stock solutions and are delivered as 1X nutrient solutions using 1:20 Dositrons (Dositron Co., Clearwater, FL, USA). Each treatment is administered for 6 minutes, 2-6 times daily using individual drip irrigators. Irrigators are controlled using a 50mL/min. emitter for delivery of 300mL to each plant per treatment. Frequency of nutrient delivery is adjusted as plant growth increases; however, between treatments the amount of nutrient conveyed to each plant is always the same. The skilled artisan will recognize that the quantity of delivered nutrient solution may need to be varied based on the plant size, greenhouse environment, and variety of corn used. These conditions are appropriate for the preferred rapid cycling maize variety. The skilled artisan will also recognize that the $KNO_3$ concentration may be varied both above and below 1mM and 6.5mM. The applicants have found these concentrations provide excellent discrimination both within and between treatments.

Table 19. Micro and macro nutrients for 20X modified Hoagland's solution

| Micro Nutients | | | Add to 1 liter of DI H20 |
|---|---|---|---|
| Constant | Entry | Ingredient | Unit |
| | 1 | 30mM H3B03 | mg |
| | 2 | 10mM MnCl2 · 4H20 | mg |
| | 3 | 10mM ZnS04 · 7H20 | mg |
| | 4 | 1mM CuS04 · 5H20 | mg |
| | 5 | 1mM Na2Mo04 H20 | mg |

| | | | | | | mMol Concentration | | | | | |
|---|---|---|---|---|---|---|---|---|---|---|---|
| NUE Assay | Entry | Ingredient | Mol Wt mg/ mMol | 20L 20x | K | P | N03 | Mg | S04 | Ca | Cl |
| | 1 | KH2P04 | 136.2 | 13.61 | 0.25 | 0.25 | | | | | |
| | 2 | MgS04 | 120.36 | 48.09 | | | | 1 | 1 | | |
| | 3 | KN03 | 101.2 | 40.44 | 1 | | 1 | | | | |

(continued)

| | | | | mMol Concentration | | | | | | | |
| NUE Assay | Entry | Ingredient | Mol Wt mg/ mMol | 20L 20x | K | P | N03 | Mg | S04 | Ca | Cl |
|---|---|---|---|---|---|---|---|---|---|---|---|
| | 4 | KCl | 74.55 | 85.71 | 3 | | | | | | 3 |
| | 5 | CaCl2 | 147.01 | 58.74 | | | | | | 1 | 2 |
| | 6 | FeS04 | 278.01 | 6.67 | | | | | 0.06 | | |
| | 7 | Sprint 330 | | 33.50 | | | | | | | |
| | 8 | 10x Micros | | 19 | | | | | | | |
| | 9 | Hydrochloric Acid | | 5 | | | | | | | |
| | | | | | 4.25 | 0.25 | 1 | 1 | 1.06 | 1 | 5 |

| | | | | mMol Concentration | | | | | | | |
| NUE Assay | Entry | Ingredient | Mol Wt mg/ mMol | 20L 20x | K | P | NO3 | Mg | SO4 | Ca | Cl |
|---|---|---|---|---|---|---|---|---|---|---|---|
| | 1 | KH2P04 | 136.2 | 13.61 | 0.25 | 0.25 | | | | | |
| | 2 | MgS04 | 120.36 | 48.09 | | | | 1 | 1 | | |
| | 3 | KN03 | 101.2 | 262.85 | 6.5 | | 6.5 | | | | |
| | 4 | KCl | 74.55 | 0 | | | | | | | |
| | 5 | CaCl2 | 147.01 | 58.74 | | | | | | 1 | 2 |
| | 6 | FeS04 | 278.01 | 6.67 | | | | | 0.06 | | |
| | 7 | Sprint 330 | | 33.5 | | | | | | | |
| | 8 | 10x Micros | | 19 | | | | | | | |
| | 9 | Hydrochloric Acid | | 5 | | | | | | | |
| | | | | | 6.75 | 0.25 | 6.5 | 1 | 1,06 | 1 | 2 |

Analysis of Maximum Biomass Between and Within Treatment Groups. Each T1 plant is imaged three times weekly using the LemnaTec Scanalyzer System. Plants are grown for 28 days.

By plotting biomass (voxel) determinations over time a growth curve is obtained and biomass is extracted as outlined in Example 1, Tables 2 and 3. Maximum biomass can be compared between positive and negative segregants within and between treatment groups. Each event is considered independently since each event will have a different level of gene expression and may have different copy number. The mean performance of all plants within a treatment group for both positive and negative segregants are shown as well. Plant growth and development was quantitatively evaluated using the LemnaTec Scanalyzer and Software as detailed in Example 1. The mean performance of the transgenic segregants for a given event is compared to the mean performance of the null segregants in each of the two nitrogen regimes. Statistically significant improvement of the mean performance of the transgenic segregants for either plant size characteristics or percentage of green colors under the limited nitrogen condition is used to identify transgenic events (and thus transgenes) which positively influence NUE at this, the T1 generation, without field evaluation.

Example 7 - Quantitative T1 Trait Assay to Identify Transgenes That Confer Enhanced Water Use Efficiency

Functional testing of transgenes that improve agronomic traits such as water use efficiency (WUE) can be evaluated. The present example takes advantage of the fact that maize plants exhibit several physiological and phenotypic traits early on in development under water limiting conditions. The measuring systems described can detect and quantify these symptoms. For example, improvements in water use efficiency can include, but are not limited to, increased biomass accumulation and leaf greenness, and photosynthesis; two traits that correlate well with the plant's ability to respond to the amount of water in the environment.

Using three dimensional imaging technologies and optionally chlorophyll fluorescence based measurements of photosynthesis, the effect of various transgenes on WUE is able to be assessed early on in the transgenic variety development process (i.e. T1 generation). This technology is able to identify genes that influence agronomic performances such as plants with enhanced traits prior to field testing. Field testing for WUE is especially challenging because the delivery of water to plants is easy to supplement, with irrigation, but hard to control (for example, rainfall provides uncontrolled water). Thus, evaluating transgenes for utility in WUE is complicated and slow at the field level. In this Example, enhancements in water use efficiency can be measured by monitoring changes in plant growth and photosynthetic performance under limiting and/or sufficient water containing environments. Applicants assess plant growth by capturing plant volume data throughout the duration of the assay using a LemnaTec Scanalyzer HTS LT-0001-2 as described in preceding Examples. In addition, optional measurement of photosynthetic capacity by analyzing chlorophyll fluorescence is performed. Applicants leverage color information captured by the imaging system in order to analyze the accumulation of specific color groups over time. For this assay, applicants have defined four color classes; green, light green, yellow, and magenta. All pixels in the plant image are assigned to one of these four classes or to the background. Differences in greenness are derived by monitoring changes in greenness of the plant under the two different water containing environments. Moreover, applicants measure chlorophyll fluorescence emissions in fully expanded leaves using a LI-6400 System (commercially available from LI-COR Biosciences of Lincoln, Nebraska USA). The skilled artisan recognizes that multiple measurements of photosynthesis can be made by different methods, e.g., fluorescence, gas exchange, or by hyperspectral spectroscopy. The LI-6400 has been found to be simple and inexpensive to use but other systems are equally applicable. This instrument captures data relating to the operating efficiency of Photo System II ($\Phi$PSII) and the maximum efficiency of open PSII reaction centers (Fv'/Fm'). $\Phi$PSII provides an estimate of the efficiency at which light is absorbed by PSII antennae and is directly related to $CO_2$ assimilation within the leaf. Fv'/Fm' is used to provide an estimate of the maximum efficiency of PSII photochemistry at a given light intensity, which is equivalent to the PSII operating efficiency if all of the PSII centers are open.

A balanced, incomplete, randomized block experiment design with stationary blocks within treatments is used in order to minimize spatial variation. The experiment is blocked by treatment and randomized by events and replicates. T1 plants are generated by crossing individual T0 hemizygous plants carrying the transgene being tested, with a recurrent male parent; optimally a small stature, rapid cycling inbred variety. The skilled artisan will recognize that T0 transformants may also be self-pollinated, or pollinated with other varieties. The applicants have found that the use of a recurrent, inbred parent, of rapid cycling characteristics, is most optimal for the conduct of this assay with minimum plant numbers. Optionally, the transgenic events to be tested can be pre-screened for expression of the transgene under study by the methods described in Example 5. Each T1 plant is grown in a classic 200 pot (volume equivalent to 1.7L) that contains a bar coded label with information about the plant's genetic identity, planting date and greenhouse location. The planting density is 8.5" center to center within a row and 9" center to center between rows. T1 seeds are sown in a mixture of 1/3 Turface, 1/3 SB300 and 1/3 sand to create a growing medium with sufficient water holding capacity. Turface is a porous ceramic soil conditioner that absorbs moisture, resists compaction and functions to aerate the soil and increase water drainage. SB300 is a medium textured, bark containing, organic soil mix (e.g. commercially available under product name Sunshine SB300 Universal professional growing mix from Sun Gro Horticulture Canada Ltd., Seba Beach Alberta CANADA). Dry weight measurements are obtained by weighing each soil-filled pot and subtracting the average weight of an empty classic 200 pot. For all soil-filled pots the dry weight should be within $\pm$10g of one another. The pots are then repeatedly hand-watered until the soil is fully saturated. Once residual water is no longer draining from the bottom of the pot, the wet soil weight is obtained by weighing each fully saturated pot and subtracting the average weight of an empty classic 200 pot. Field capacity (FC) or the water-holding capacity of the soil mixture can then be calculated by subtracting the average wet soil weight from the average dry soil weight and multiplying the outcome by a factor of 100 in order to express the measure as a percent. Seeds are then sown at a uniform depth of 1.5" from the surface of the growing medium and lightly hand-watered to settle the soil mixture around the seed. The average weight for a representative sample of pots per treatment is recorded daily throughout the duration of the assay in order to monitor water loss and calculate subsequent field capacity. Following planting, the soil is allowed to dry to 50% FC at which point the soil is maintained until administration of treatments. A modified Hoagland's nutrient solution containing 6.5mM $KNO_3$ is used for watering (Table 10). Emergence data is recorded for each germinating seed, whereupon plants begin being imaged three times weekly in order to capture data relating to plant growth and chlorophyll content. Seedlings segregating 1:1 are assayed for the presence of a marker gene that is contained within the T-DNA in order to identify transgene positive vs. null segregants (outlined in Example 3). Thirty plants consisting of 15 positive plants and 15 negative plants from each event are required to have an 80% power of detecting a 20% increase in biomass accumulation and/or photosynthetic performance per treatment. A half teaspoon of Osmocote (Scotts Co., Marysville, OH), a slow release fertilizer, is applied at the V2-V3 stage of development.

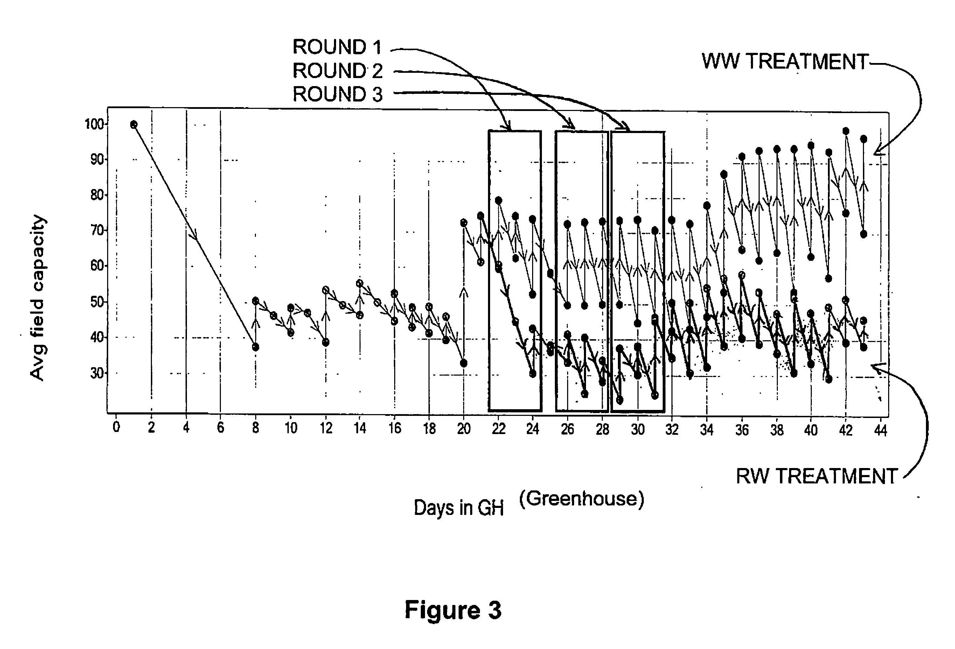

Two treatments are applied at approximately the V5-V6 stage of development during the WUE Assay; a water limiting or reduced watered treatment (RW treatment) and a well watered treatment (WW treatment, Figure 3). Plants in the WW treatment are brought up to 75% FC, while plants in the RW treatment are no longer watered until reaching 25% FC in order to impose a chronic drought stress. Chronic drought symptoms include, but are not limited to, changes in leaf color and decreased biomass accumulation. Plants in the WW treatment continue to be watered with the 6.5mM

$KNO_3$ nutrient solution, while plants in the RW treatment are watered with a higher nitrate containing Hoagland's nutrient solution of 9mM $KNO_3$ (Table 10). Both nutrient solutions are maintained as 20X stock solutions and are delivered as 1X nutrient solutions using 1:20 dositrons. Each treatment is administered once daily at approximately the same time using individual drip irrigators. Irrigators are controlled using 50mL/min. emitters. Once plants in the RW treatment reached 25% FC, acute drought symptoms are observed. Acute drought symptoms include, but are not limited to, leaf graying, leaf flaccidity, and leaf rolling. Once the RW treatment shows symptoms of acute drought stress, chlorophyll fluorescence measurements are obtained for all plants in both treatments. In addition, the acute drought-stressed plants are imaged with the LemnaTec system in order to asses the severity of leaf rolling, which manifests as a change in plant volume, between and within segregating events. Once measurements have been taken, the plants are brought up to 40% FC. Typically, plants subjected to the RW treatment incur three rounds of acute drought stress and recovery (Figure 3). Finally, as the plant's age and growth increases, the field capacity for both the WW treatment and the RW treatment can be raised to 100% and 50% respectively.

**Table 10**. Micro and macro nutrients for 20X modified Hoagland's solution

| Micro Nutients | | | | Add to 1 liter of DI H2O | |
|---|---|---|---|---|---|
| Constant | Entry | Ingredient | | Unit | 10x |
| | 1 | 30mM H3BO3 | | mg | 1854 |
| | 2 | 10mM MnCl2 · 4H2O | | mg | 1980 |
| | 3 | 10mM ZnSO4 · 7H2O | | mg | 2874 |
| | 4 | 1mM CuSO · 5H2O | | mg | 250 |
| | 5 | 1mM Na2MoO4 H2O | | mg | 242 |

| WUE Assay | Entry | Ingredient | Mol Wt mg/ mMol | 20L 16x | 20L 20x | K | P | NO3 | Mg | SO4 | NH4 | Ca |
|---|---|---|---|---|---|---|---|---|---|---|---|---|
| | | | | | | | | | mMol Concentration | | | |
| | 1 | KH2PO4 | 136.00 | | 27.17 | 0.5 | 0.5 | | | | | |
| WW Trt | 2 | KN03 | 101.00 | | 181.61 | 4.5 | | 4.5 | | | | |
| | 3 | MgSO4 (Epson salt) | 120.00 | | 95.90 | | | | 2 | 2 | | |
| | 4 | Sprint330 | | | 33.50 | | | | | | | |
| | 5 | Ca(NO3)2· 4H20 | 236 | | 94.30 | | | 2 | | | | 1 |
| | 6 | 10x Micros DI Water | | | 19 | | | | | | | |
| | 7 | Sulfuric Acid | | | 5 | | | | | | | |
| | | | | | | 5 | 0.5 | 6.5 | 2 | 2 | 0 | 1 |

| WUE Assay | Entry | Ingredient | Mol Wt mg/ mMol | 20L 16x | 20L 20x | K | P | NO3 | Mg | SO4 | NH4 | Ca |
|---|---|---|---|---|---|---|---|---|---|---|---|---|
| | | | | | | | | | mMol Concentration | | | |
| | 1 | KH2PO4 | 136.00 | | 21.17 | 0.5 | 0.5 | | | | | |
| RW Trt | 2 | KNO3 | 101.00 | | 181.61 | 4.5 | | 4.5 | | | | |
| | 3 | MgSO4 (Epson salt) | 120.00 | | 95.90 | | | | 2 | 2 | | |

(continued)

| | | | | | | mMol Concentration | | | | | | |
|---|---|---|---|---|---|---|---|---|---|---|---|---|
| WUE Assay | Entry | Ingredient | Mol Wt mg/ mMol | 20L 16x | 20L 20x | K | P | NO3 | Mg | SO4 | NH4 | Ca |
| | 4 | NH4NO3 | 80.04 | | 79.96 | | | 2.5 | | | 2.5 | |
| | 5 | Sprint330 | | | 33.50 | | | | | | | |
| | 6 | Ca(NO3)2 · 4H2O | 236 | | 94.30 | | | 2 | | | | 1 |
| | 1 | 10x Micros DI Waler | | | 19 | | | | | | | |
| | 8 | Sulfuric Acid | | | 5 | | | | | | | |
| | | | | | | 5 | 0.5 | 9 | 2 | 2 | 2.5 | 1 |

By plotting biomass (voxel) determinations over time, a growth curve is obtained and biomass is extracted as outlined in Example 1, Tables 2 and 3. Maximum biomass can be compared between positive and negative segregants within and between treatment groups. Photosynthetic measurements obtained with the Li-Cor and plant volume measurements obtained with the imaging system can also be compared between positive and negative segregants and within and between treatment groups. Each event is considered independently since each event will have a different level of gene expression and may have different copy number. The mean performance of all plants within a treatment group for both positive and negative segregants are shown as well. Plant growth and development was quantitatively evaluated using the LemnaTec Scanalyzer and Software as detailed in Example 1. The mean performance of the transgenic segregants for a given event is compared to the mean performance of the null segregants in each of the two water treatments and also after the acute stress treatment. Statistically significant improvement of the mean performance of the transgenic segregants under RW condition, or during the acute treatment phase, as compared to the null segregants, in any of the measurements is used to identify transgenic events (and thus transgenes) which positively influence WUE at this, the T1 generation, without field evaluation.

Options and Alternatives

It will be appreciated by those skilled in the art that the invention can take on different forms and embodiments over and above those described herein. For example, variations obvious to those skilled in the art will be included within the invention.

A specific example is as follows. A Dixon's Q-test (Rorabacher 1991, Analytical Chemistry Vol. 63 No. 2 pp. 129-146) can be applied to the outlier values to determine if those individual transgenic plants (events) can be excluded from the mean at varying levels of confidence (e.g. 90%, 95% etc). Plants that can be excluded based on the Dixon's Q test at a given significance level are considered as potential leads. However, other methods are possible.

**Claims**

1. A method for the rapid analysis of the effect of a transgene of interest on a selected phenotypic parameter in corn, the method comprising the steps of:

   providing a population of transgenic corn plants grown in an environmentally controlled greenhouse environment;
   providing a light spectrum digital analyzing device;
   using the light spectrum digital analyzing device to take original reflected light images of each plant and then analyze those images to determine a value for the phenotypic parameter in each plant; and
   calculating a mean or median value of the phenotypic parameter and the biological variance for the population of transgenic plants;
   whereby any statistically significant difference between the phenotypic parameter value for a single member of the population of transgenic plants and the mean or median value for the population of transgenic plants may be considered attributable to the transgene of interest.

**2.** The method of claim 1 wherein the light spectrum digital analyzing device has an instrumental variance below about 5%.

**3.** The method of claim 1 or 2 wherein the population of transgenic plants is a fast cycling uniform maize line.

**4.** The method of any one of the preceding claims wherein each plant within the population of transgenic plants is associated with a machine-readable identification number containing information about the plant's identity and greenhouse location.

**5.** The method of claim 4 wherein the phenotypic parameter value for each plant is automatically associated with that plant's identity.

**6.** The method of any one of the preceding claims wherein the selected phenotypic parameter is a yield component trait.

**7.** The method of claim 6 wherein the yield component trait is selected from the group consisting of leaf angle, anthesis-silking interval (ASI), staygreen ability, early growth rate, overall growth rate, maximum biomass, total biomass, nitrogen use efficiency, and water use efficiency.

**8.** The method of any one of the preceding claims wherein the transgenic plants are grown in a randomized block planting pattern.

**9.** The method of any one of the preceding claims wherein the light spectrum digital analyzer takes reflected light images of each plant from at least three angles under controlled lighting conditions in an imaging analysis area.

**10.** The method of claim 9 in which the plants are transported through the image analysis area on a conveyor system.

**11.** The method of claim 10 wherein each plant is rotated while it is in the image analysis area so that two of the at least three images are obtained with a single camera.

**12.** The method of claim 9 in which the image analysis area is covered by an opaque housing to prevent light from entering the image analysis area.

**13.** The method of any one of the preceding claims further comprising the steps of:

> providing a computer data base accessible by a user computer; and
> automatically storing the original reflected light images and analyzed images in the computer database.

**14.** The method of any one of the preceding claims wherein the selected phenotypic parameter is a pollen shed date, and the imaging and analysis step includes the steps of:

> analyzing each plant stem for color value;
> comparing this color value to a color classification system in which certain colors are considered indicative of active male anthers;
> determining whether an active male anther is present on each plant based on the plant stem color value analysis; and
> calculating a pollen shed date for each plant.

**15.** A method for the high throughput analysis of a yield component trait in transgenic corn, the method comprising the steps of:

> providing recipient maize plant cells from a plant line for transformation;
> obtaining a plurality of nucleotide vectors;
> introducing the plurality of nucleotide vectors into the recipient maize plant cells to create a plurality of transgenic plants;
> allowing the plurality of transgenic plants and, optionally, a plurality of control plants, to grow in an environmentally controlled greenhouse environment, each plant being associated with a machine-readable label that distinguishes the plant from other plants;
> analyzing each transgenic plant and, optionally, each control plant, for the yield component trait using quantitative, non-destructive imaging technology and associating the yield component trait with the machine-readable information number for that plant; and

comparing the yield component trait for one or more transgenic plants with the mean or median yield component trait of transgenic plants, or optionally, with the mean or median yield component trait for the control plants, to determine the effect of the nucleotide vectors on the yield component trait.

**16.** The method of claim 15 wherein the yield component trait is selected from the group consisting of leaf angle, anthesis-silking interval (ASI), staygreen ability, early growth rate, total biomass, partial biomass, nitrogen use efficiency, and water use efficiency.

**17.** The method of claim 16 wherein the yield component trait is total biomass and the quantitative, non-destructive imaging technology comprises a light spectrum digital analyzer and the analyzing step comprises taking reflected light images of each plant from three angles under controlled lighting conditions.

**18.** The method of any one of claims 15 to 17 wherein each machine-readable identification number is associated with a unique, physical greenhouse location.

**19.** The method of any one of claims 15 to 18 wherein the transgenic plants and optional control plants are grown in a randomized block planting pattern.

**20.** The method of any one of claims 15 to 19 in which, during the analyzing step, at least three reflected light images of the plant from three different angles are obtained using an imaging analyzer located in an image analysis area.

**21.** The method of claim 20 in which the plants are transported through the image analysis area on a conveyor system.

**22.** The method of claim 21 comprising the further step of rotating the plant while it is in the image analysis area so that two of the at least three images are obtained with a single camera.

**23.** The method of claim 22 in which the image analysis area is covered by a housing to prevent light from entering the image analysis area.

**24.** The method of claim 23 wherein the imaging analyzer is located in the greenhouse environment.

**25.** The method of any one of claims 15 to 24 wherein the plant line that is the source of the recipient maize plant cells is a fast cycling, highly transformable line.

**26.** A method for the rapid analysis of the effect of a gene of interest on a selected phenotypic parameter in corn, the method comprising the steps of:

providing a population of T0 transgenic corn plants possessing the gene of interest and grown in an environmentally controlled greenhouse environment;

using a light spectrum digital analyzing device having an instrumental variance below about 5% to take original reflected light images of each plant and then analyze those images to obtain phenotypic data for each plant; and

performing a statistical analysis of the phenotypic data to analyze the effect of the gene of interest on the selected phenotypic parameter.

**27.** The method of claim 26 including as a first step introducing a transgene construct possessing at least one gene of interest into recipient maize cells to produce an event population that grows into the population of T0 transgenic corn plants.

**28.** The method of claim 27 wherein the statistical analysis comprises the step of:

calculating a mean or median value of the phenotypic parameter for a single transgene construct and for the population of transgenic plants;

whereby any statistically significant difference between the mean or median value of the phenotypic parameter value for the single transgene construct and the mean or median value of the phenotypic parameter for the population of transgenic plants may be considered attributable to the transgene of interest.

**29.** A method for the rapid analysis of the effect of a gene of interest on a selected phenotypic parameter in corn, the method comprising the steps of:

providing a population of T0 transgenic corn plants possessing the gene of interest and grown in an environmentally controlled greenhouse environment;

pollinating the T0 transgenic corn plants with a recurrent parent to obtain T1 plants;

assaying the T1 plants to identify plants possessing the gene of interest;

using a light spectrum digital analyzing device having an instrumental variance below about 5% to take original reflected light images of each T1 plant and then analyze those images to obtain phenotypic data for each plant; and

performing a statistical analysis of the phenotypic data to analyze the effect of the gene of interest on the selected phenotypic parameter.

**30.** The method of any one of claims 1 to 14 wherein if there is a statistically significant difference, an electrical signal is generated indicating that the difference is attributable to the transgene of interest.

**31.** The method of any one of claims 15 to 25 further comprising generating an electrical signal indicating the effect of the nucleotide vectors on the yield component trait.

**32.** The method of any one of claims 26 to 29 further comprising generating an electrical signal indicative of the result of the statistical analysis.

Figure 1

Figure 2

Figure 3

**European Patent Office**

**EUROPEAN SEARCH REPORT**

Application Number

EP 07 25 0405

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/059562 A (UNIV COLORADO RES [US]; MEDFORD JUNE IRIS [US]; PARSONS RONALD LEE [US]) 1 August 2002 (2002-08-01) <br><br> * page 5, line 24 - page 9, line 26; claims 29-32,42; figures 1-4; example 1 * <br> * page 11, line 20 - page 16, line 26 * <br> * page 32, line 25 - page 35, line 7 * <br> ----- | 1-8,10, 12,13, 15,16, 18,19, 21,25-32 | INV. <br> C12N15/82 <br> A01H1/04 <br> G06T7/60 |
| A | WO 01/83697 A (EXELIXIS PLANT SCIENCES INC [US]) 8 November 2001 (2001-11-08) <br> * page 37 - page 39 * <br> ----- | 1-32 | |
| P,X | REUZEAU CHRISTOPHE ET AL: "Traitmill (TM): a functional genomics platform for the phenotypic analysis of cereals" PLANT GENETIC RESOURCES CHARACTERIZATION AND UTILIZATION, vol. 4, no. 1, April 2006 (2006-04), pages 20-24, XP002434027 ISSN: 1479-2621(print) 1479-263x(ele * the whole document * <br> ----- | 1,2,4, 6-8,10, 13,15, 16,18, 19,21, 26-32 | |
| | | | TECHNICAL FIELDS SEARCHED (IPC) <br><br> C12N <br> A01H <br> G06T |

The present search report has been drawn up for all claims

| Place of search | Date of completion of the search | Examiner |
|---|---|---|
| The Hague | 16 May 2007 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 25 0405

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2007

| Patent document cited in search report | | Publication date | Patent family member(s) | | Publication date |
|---|---|---|---|---|---|
| WO 02059562 | A | 01-08-2002 | AU | 2002251830 A1 | 06-08-2002 |
| | | | EP | 1366349 A2 | 03-12-2003 |
| WO 0183697 | A | 08-11-2001 | AU | 5926601 A | 12-11-2001 |
| | | | CA | 2406104 A1 | 08-11-2001 |
| | | | EP | 1279120 A2 | 29-01-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006055487 A2
- WO 0183697 A2
- US 20040128712 A1
- US 20030221212 A
- US 20040122592 A

**Non-patent literature cited in the description**

- **BOYES et al.** Growth Stage-Based Phenotypic Analysis of Arabidopsis: A Model for High Throughput Functional Genomics in Plants. *The Plant Cell,* July 2001, vol. 13, 1499-1510
- **MENDA et al.** In silico screening of a saturated mutation library of tomato. *The Plant Journal,* 2004, vol. 38, 861-872
- **RORABACHER.** *Analytical Chemistry,* 1991, vol. 63 (2), 129-146